(19) 

**Europäisches Patentamt**
**European Patent Office**
**Office européen des brevets**

(11) **EP 4 736 768 A1**

(12) **EUROPEAN PATENT APPLICATION**
published in accordance with Art. 153(4) EPC

(43) Date of publication:
**06.05.2026 Bulletin 2026/19**

(21) Application number: **24851834.2**

(22) Date of filing: **06.08.2024**

(51) International Patent Classification (IPC):
***A61B 5/353*** (2021.01)

(52) Cooperative Patent Classification (CPC):
**A61B 5/353**

(86) International application number:
**PCT/JP2024/028015**

(87) International publication number:
**WO 2025/033410 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.08.2023 JP 2023130329**

(71) Applicants:
• **OMRON Corporation**
 **Kyoto-shi, Kyoto 600-8530 (JP)**
• **Omron Healthcare Co., Ltd.**
 **Muko-shi, Kyoto 617-0002 (JP)**

(72) Inventors:
• **KUBO, Mitsuaki**
 **Kyoto-shi, Kyoto 600-8530 (JP)**
• **KOIZUMI, Masayuki**
 **Kyoto-shi, Kyoto 600-8530 (JP)**
• **WANG, Danni**
 **Kyoto-shi, Kyoto 600-8530 (JP)**
• **KIMURA ISHIDA, Yui**
 **Kyoto-shi, Kyoto 600-8530 (JP)**
• **FUJII, Kenji**
 **Muko-shi, Kyoto 617-0002 (JP)**
• **KAWABATA, Yasuhiro**
 **Muko-shi, Kyoto 617-0002 (JP)**

(74) Representative: **Isarpatent**
**Patent- und Rechtsanwälte PartG mbB**
**Friedrichstraße 31**
**80801 München (DE)**

(54) **BIOSIGNAL PROCESSING DEVICE, BIOSIGNAL MEASURING DEVICE, AND BIOSIGNAL PROCESSING METHOD**

(57) A plurality of heartbeat waveforms extracted from time-series data of an ECG signal are divided into a plurality of heartbeat waveform groups including at least a first heartbeat waveform group and a second heartbeat waveform group, a first representative P wave interval waveform and a second representative P wave interval waveform, which are typical P wave interval waveforms, are acquired from the first heartbeat waveform group and the second heartbeat waveform group, respectively, and when a similarity between the first representative P wave interval waveform and the second representative P wave interval waveform is higher than a threshold value, it is determined that a P wave is included in the time-series data of the ECG signal.

FIG. 12

EP 4 736 768 A1

**Description**

TECHNICAL FIELD

The present invention relates to a biosignal processing device, a biosignal measuring device, and a biosignal processing method.

BACKGROUND OF INVENTION

Atrial fibrillation (AF) is a type of arrhythmia, and is a disease in which the atria fibrillate rapidly due to abnormal electrical signals generated from sites other than the sinoatrial node, and the pulse (heartbeat interval) becomes irregular. Atrial fibrillation not only causes palpitations and shortness of breath, which can interfere with daily life, but also has the potential to cause serious diseases such as stroke and heart failure. Therefore, early detection and appropriate treatment of atrial fibrillation are desired.

It is known that two characteristics, "the irregularity of the heartbeat interval" and "P wave disappearance", appear in an electrocardiogram (ECG) during a seizure of atrial fibrillation. Therefore, in order to realize automatic detection of atrial fibrillation by signal processing, it is necessary to accurately evaluate the irregularity of the heartbeat interval and detect a P wave (determine P wave presence/absence) from the time-series data of the ECG signal. Here, since the irregularity of the heartbeat interval is captured as the variation of an RRI (RR interval; a time from the peak of an R wave to the peak of a next R wave) in the ECG signal, the irregularity can be evaluated relatively easily and accurately. However, it is quite difficult to accurately determine the P wave presence/absence. This is because the amplitude of the P wave is much smaller than that of the R wave and is easily contaminated by noise (it is difficult to distinguish the P wave from the noise). In addition, the fact that there are individual differences in the waveform (amplitude, shape, orientation (polarity), and the like) of the P wave also increases the difficulty of detecting the P wave.

PTL 1 discloses an idea of generating a template of P waves based on an ECG signal, extracting only waveforms having a high cross-correlation with the template from heartbeat waveforms of the ECG signal, and averaging the waveforms, thereby detecting P waves with high accuracy.

CITATION LIST

PATENT LITERATURE

PTL 1: US 5840038

NON-PATENT LITERATURE

NPL 1: Saclova, L., Nemcova, A., Smisek, R. et al., "Reliable P wave detection in pathological ECG signals", Sci Rep 12, 6589 (2022).

SUMMARY

TECHNICAL PROBLEM

In a known detection algorithm, there are cases where an erroneous detection of "P wave present" occurs even though the P wave is not present, or conversely, an erroneous determination of "P wave absent" occurs even though the P wave is present. When such erroneous detection or erroneous determination occurs, the subsequent determination of atrial fibrillation is affected, and the detection accuracy or reliability of atrial fibrillation is significantly reduced.

The present invention has been made in view of the above circumstances, and an object thereof is to provide a novel technique for determining presence of a P wave with high reliability from time-series data of an EGG signal by signal processing.

SOLUTION TO PROBLEM

The present disclosure includes a biosignal processing device including: an acquisition unit that acquires time-series data of an ECG signal measured from a user; and a P wave determination unit that extracts a first P wave interval waveform from a first P wave interval set with reference to a time point of a first R wave in the time-series data of the ECG signal and extracts a second P wave interval waveform from a second P wave interval set with reference to a time point of a second R wave different from the first R wave, and when a similarity between the first P wave interval waveform and the second P wave interval waveform is higher than a threshold value, determines that a P wave is included in the first P wave interval and the second P wave interval.

The present disclosure includes a biosignal processing device including: an acquisition unit that acquires time-series data of an ECG signal measured from a user; and a P wave determination unit that extracts at least a first heartbeat waveform group and a second heartbeat waveform group from the time-series data of the ECG signal, extracts waveforms of P wave intervals each set with reference to a time point of an R wave from respective heartbeat waveforms of the first heartbeat waveform group and extracts waveforms of P wave intervals each set with reference to a time point of an R wave from respective heartbeat waveforms of the second heartbeat waveform group, generates a first representative P wave interval waveform, based on a waveform group of a plurality of P wave intervals extracted from the first heartbeat waveform group and generates a second representative P wave interval waveform, based on a waveform group of a plurality of P wave intervals extracted from the second heartbeat waveform group, and when a similarity between the first representative P wave interval waveform and the second representative P wave interval waveform is higher than a threshold value, determines that a P wave is included in the first heartbeat waveform group and the second heartbeat waveform group.

The present disclosure includes a biosignal processing device including: an acquisition unit that acquires time-series data of an ECG signal measured from a user; a candidate detection unit that detects a P wave candidate from the time-series data of the ECG signal; and a P wave determination unit that, in a case where the P wave candidate is detected by the candidate detection unit, divides a plurality of heartbeat waveforms extracted from the time-series data of the ECG signal into a plurality of heartbeat waveform groups including at least a first heartbeat waveform group and a second heartbeat waveform group, acquires a first representative P wave interval waveform and a second representative P wave interval waveform, which are typical P wave interval waveforms, from the first heartbeat waveform group and the second heartbeat waveform group, respectively, and when a similarity between the first representative P wave interval waveform and the second representative P wave interval waveform is higher than a threshold value, determines that a P wave is included in the time-series data of the ECG signal.

When the similarity between the first representative P wave interval waveform and the second representative P wave interval waveform is equal to or less than the threshold value, the P wave determination unit may determine that whether the P wave is included in the time-series data of the ECG signal is indeterminable.

The P wave determination unit may allocate, among the plurality of heartbeat waveforms extracted from the time-series data of the ECG signal, a heartbeat waveform extracted in a first half into the first heartbeat waveform group and a heartbeat waveform extracted in a second half into the second heartbeat waveform group. Alternatively, the P wave determination unit may alternately or randomly select, from among the plurality of heartbeat waveforms extracted from the time-series data of the ECG signal, a heartbeat waveform to be allocated to the first heartbeat waveform group and a heartbeat waveform to be allocated to the second heartbeat waveform group.

The P wave determination unit may divide the plurality of heartbeat waveforms extracted from the time-series data of the ECG signal into the first heartbeat waveform group and the second heartbeat waveform group such that the number of heartbeat waveforms of the first heartbeat waveform group and the number of heartbeat waveforms of the second heartbeat waveform group are equal to each other.

The P wave determination unit may extract waveforms of P wave intervals each set with reference to a time point of an R wave from respective heartbeat waveforms of the first heartbeat waveform group, and acquire, as the first representative P wave interval waveform, an average, a median value, or a mode value of waveforms of a plurality of P wave intervals extracted from the first heartbeat waveform group, and extract waveforms of P wave intervals each set with reference to a time point of an R wave from respective heartbeat waveforms of the second heartbeat waveform group, and acquires, as the second representative P wave interval waveform, an average, a median value, or a mode value of waveforms of a plurality of P wave intervals extracted from the second heartbeat waveform group.

The P wave determination unit may evaluate the similarity between the first representative P wave interval waveform and the second representative P wave interval waveform by cross-correlation between the first representative P wave interval waveform and the second representative P wave interval waveform.

The candidate detection unit may generate a representative heartbeat waveform, which is a typical heartbeat waveform, from a plurality of heartbeat waveforms extracted from the time-series data of the ECG signal, and detect a P wave candidate from the representative heartbeat waveform.

The candidate detection unit may extract a feature amount from a P wave interval set with reference to a time point of an R wave in the representative heartbeat waveform, and when the feature amount satisfies a predetermined condition, determine that the P wave candidate is present.

The candidate detection unit may select, from the time-series data of the ECG signal, data of a low-noise interval in which a degree of noise is lower than a predetermined level, and generate the representative heartbeat waveform by using a plurality of heartbeat waveforms extracted from the data of the low-noise interval.

When the number of heartbeat waveforms extracted from the data of the low-noise interval is less than a predetermined number, the P wave determination unit may determine that whether the P wave is included in the time-series data of the ECG signal is indeterminable.

When the P wave candidate is not detected by the candidate detection unit, the P wave determination unit may evaluate a degree of smoothness of a P wave interval set with reference to a time point of an R wave in the representative heartbeat waveform, determine, in a case where the degree of smoothness is equal to or greater than a predetermined value, that the P wave is not included in the time-series data of the ECG signal, and determine, in other cases, that whether the P wave is included in the time-series data of the ECG signal is indeterminable.

The present disclosure includes a biosignal measuring device including: an ECG sensor that is configured to be worn on a limb of a user; and the biosignal processing device that processes an ECG signal measured by the ECG sensor.

The present disclosure includes a biosignal processing method performed by a biosignal processing device, the biosignal processing method including: acquiring time-series data of an ECG signal measured from a user; and extracting a first P wave interval waveform from a first P wave interval set with reference to a time point of a first R wave in the time-series data of the ECG signal and extracting a second P wave interval waveform from a second P wave interval set with reference to a time point of a second R wave different from the first R wave, and when a similarity between the first P wave interval waveform and the second P wave interval waveform is higher than a threshold value, determining that a P wave is included in the first P wave interval and the second P wave interval.

The present disclosure includes a biosignal processing method performed by a biosignal processing device, the biosignal processing method including: acquiring time-series data of an ECG signal measured from a user; and extracting at least a first heartbeat waveform group and a second heartbeat waveform group from the time-series data of the ECG signal, extracting waveforms of P wave intervals set with reference to a time point of an R wave from respective heartbeat waveforms of the first heartbeat waveform group and extracting waveforms of P wave intervals each set with reference to a time point of an R wave from respective heartbeat waveforms of the second heartbeat waveform group, generating a first representative P wave interval waveform, based on a waveform group of a plurality of P wave intervals extracted from the first heartbeat waveform group and generating a second representative P wave interval waveform, based on a waveform group of a plurality of P wave intervals extracted from the second heartbeat waveform group, and when a similarity between the first representative P wave interval waveform and the second representative P wave interval waveform is higher than a threshold value, determining that a P wave is included in the first heartbeat waveform group and the second heartbeat waveform group.

The present disclosure includes a biosignal processing method performed by a biosignal processing device, the biosignal processing method including: acquiring time-series data of an ECG signal measured from a user; detecting a P wave candidate from the time-series data of the ECG signal; and in a case where the P wave candidate is detected, dividing a plurality of heartbeat waveforms extracted from the time-series data of the ECG signal into a plurality of heartbeat waveform groups including at least a first heartbeat waveform group and a second heartbeat waveform group, acquiring a first representative P wave interval waveform and a second representative P wave interval waveform, which are typical P wave interval waveforms, from the first heartbeat waveform group and the second heartbeat waveform group, respectively, and when a similarity between the first representative P wave interval waveform and the second representative P wave interval waveform is higher than a threshold value, determining that a P wave is included in the time-series data of the ECG signal.

The present invention may be regarded as a biosignal processing device including at least a part of the above configuration, or may be regarded as a biosignal measuring device or an electrocardiogram measuring device that includes an ECG sensor and the biosignal processing device. Alternatively, the present invention may be regarded as an atrial fibrillation detection device, an atrial fibrillation recording device, or an atrial fibrillation monitoring device. Further, the present invention can be regarded as a biosignal processing method including at least a part of the above processing, a method of controlling a device, or a program for implementing such a method and a non-transitory recording medium on which the program is recorded. Note that the present invention can be configured by combining each of the above-described configurations and processing operations to the extent possible.

ADVANTAGEOUS EFFECTS OF INVENTION

According to the present invention, it is possible to determine the presence of the P wave with high reliability from the time-series data of the EGG signal by signal processing.

BRIEF DESCRIPTION OF THE DRAWINGS

[FIG. 1] FIG. 1 is a diagram illustrating a state in which a biosignal measuring device is worn on an upper arm.
[FIG. 2] FIG. 2 is a plan view of the biosignal measuring device.
[FIG. 3] FIG. 3 is a perspective view of the biosignal measuring device.
[FIG. 4] FIG. 4 is a block diagram illustrating a hardware configuration of the biosignal measuring device.

[FIG. 5] FIG. 5 is a block diagram illustrating a functional configuration of the biosignal measuring device.

[FIG. 6] FIG. 6 is a flowchart illustrating a flow of measurement processing.

[FIG. 7] FIG. 7 is a diagram illustrating an example of an electrode pair and an ECG signal.

[FIG. 8] FIG. 8 is a diagram illustrating a waveform of an ECG signal and heartbeat information.

[FIG. 9] FIG. 9 is a block diagram illustrating a functional configuration of a P wave detection unit.

[FIG. 10] FIG. 10 is a flowchart illustrating a flow of P wave detection processing.

[FIG. 11] FIG. 11 is a diagram illustrating P wave candidate detection.

[FIG. 12] FIG. 12 is a diagram illustrating an algorithm of P wave presence determination processing.

[FIG. 13] FIG. 13 is a diagram illustrating an algorithm of the P wave presence determination processing according to a modified example.

DESCRIPTION OF EMBODIMENTS

<Application Example>

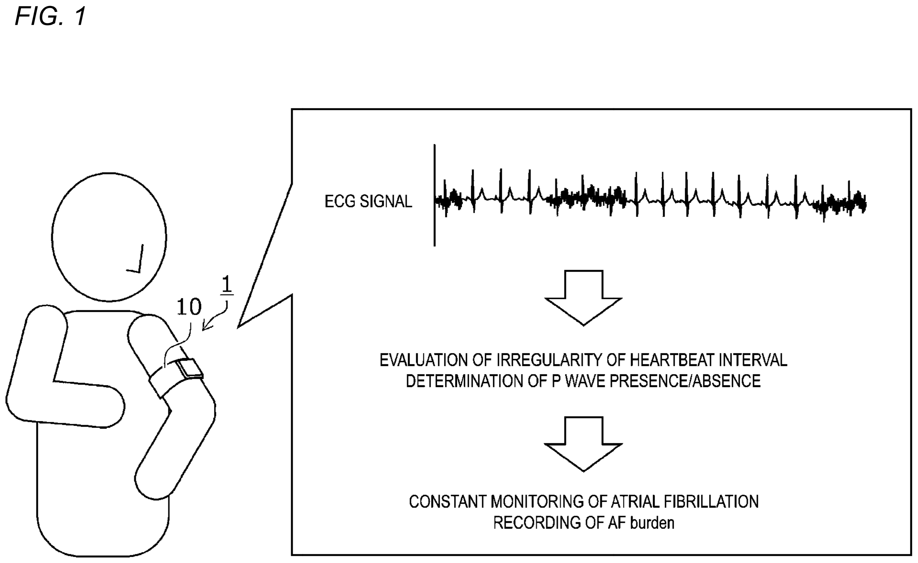

An application example of the present invention will be described with reference to FIG. 1.

A biosignal measuring device 1 is a portable measuring device that is used by being worn on a limb, and is capable of monitoring an ECG signal (electrocardiogram signal) for 24 hours. The biosignal measuring device 1 includes an ECG sensor for measuring an ECG signal, and a biosignal processing device for analyzing time-series data of the ECG signal measured by the ECG sensor and automatically detecting atrial fibrillation.

At the time of measurement, a user wears the biosignal measuring device 1 by wrapping a band 10 around a measurement site. Examples of measurement sites where the band 10 can be worn include an upper limb (an upper arm, a forearm, a wrist, a hand, and a finger), a lower limb (a thigh, a lower leg, an ankle, a foot, and a toe), and the like, and the measurement site is appropriately selected depending on the type of a biosignal to be measured, a measurement algorithm, and the like.

As described above, in order to improve the detection accuracy and reliability of atrial fibrillation, it is necessary to determine the P wave presence/absence with high accuracy. In this regard, the biosignal measuring device 1 first detects a P wave candidate from an ECG signal using a simple algorithm, and then performs detailed P wave determination processing to make a final determination as to the P wave presence/absence. The P wave determination processing may include a "P wave presence determination" that is executed when the P wave candidate is detected and a "P wave absence determination" that is executed when the P wave candidate is not detected. In the "P wave presence determination", two waveforms in a P wave interval are acquired from the time-series data of the ECG signal in a target period, and the determination of "P wave present" is made in a case where a similarity between the two waveforms is higher than a threshold value, and a determination of "P wave presence/absence is indeterminable" is made in other cases. In the "P wave absence determination", the degree of smoothness of the P wave interval is obtained from the time-series data of the ECG signal in the target period, and a determination of "P wave absent" is made in a case where the degree of smoothness is high, and the determination of "P wave presence/absence is indeterminable" is made in other cases. In the present specification, the "P wave interval" is a time range that includes a time range in which a P wave is generated prior to an R wave when the heartbeat is normal, and does not include other electrocardiogram waveforms (for example, a Q wave and a T wave) in proximity.

By this processing, the determination of "P wave present" is made in a case where a P wave is included in the ECG signal of the target period with high accuracy, and the determination of "P wave absent" is made in a case where a P wave is not included in the ECG signal of the target period with high accuracy. Then, when it is difficult to make a determination with high accuracy due to the influence of noise or the like, a determination result of "P wave presence/absence is indeterminable" is output.

For example, when an ECG signal labeled as "P wave presence/absence is indeterminable" is excluded and only an ECG signal labeled as "P wave present" or "P wave absent" is used for detection of atrial fibrillation, it can be expected that the detection accuracy and reliability of atrial fibrillation can be improved as compared with the related art.

Hereinafter, as an embodiment of the present invention, a specific configuration example in a case where the present invention is applied to an upper arm electrocardiographic device will be described.

<Embodiments>

(Device Configuration)

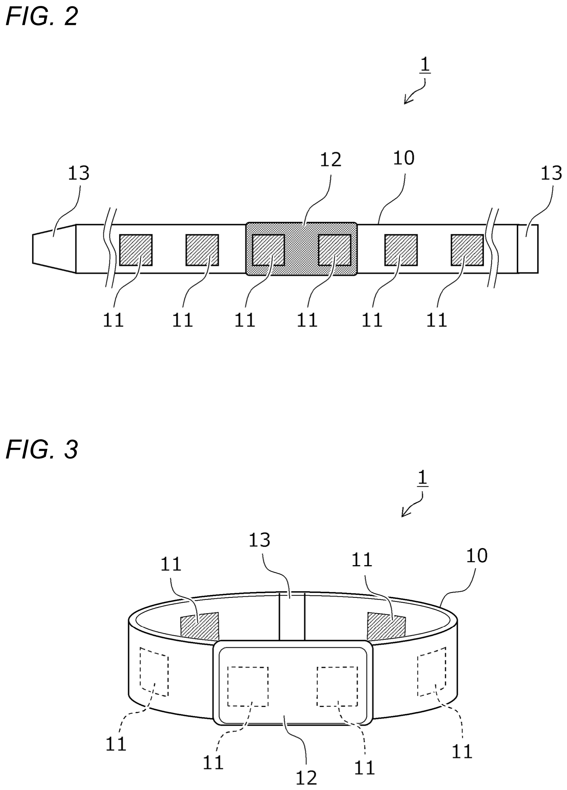

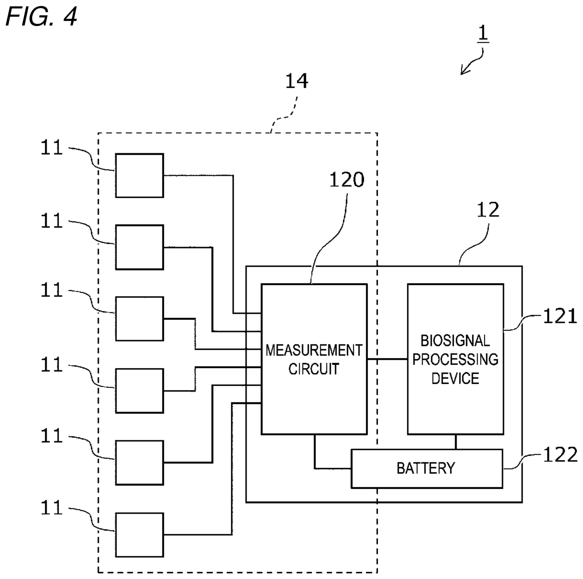

An embodiment of the present invention will be described with reference to FIGS. 1 to 4. FIG. 1 is a diagram illustrating a state in which the biosignal measuring device 1 is worn on an upper arm, FIG. 2 is a plan view of the biosignal measuring device 1, FIG. 3 is a perspective view of the biosignal measuring device 1, and FIG. 4 is a block diagram illustrating a hardware configuration of the biosignal measuring device 1.

The biosignal measuring device 1 of the present embodiment is an upper arm electrocardiographic device that is worn on an upper arm (preferably, a left upper arm close to the heart) of a user and is used to measure an electrocardiogram (ECG) signal as a biosignal.

The biosignal measuring device 1 includes, as main components, the band 10, a plurality of electrodes 11 fixed to the band 10, and a control body 12 fixed to the band 10.

The band 10 is a member (fixing member) for fixing the electrodes 11 in a state in which the electrodes are pressed against a living body. In the present embodiment, a belt-shaped band 10 made of a flexible and pliable material (for example, chemical fiber, silicon, leather, or the like) is used. A fixing mechanism 13 is provided at a longitudinal end portion of the band 10. As illustrated in FIGS. 1 and 3, the biosignal measuring device 1 can be worn on the upper arm by forming the band 10 into a loop shape and fastening the band with the fixing mechanism 13. The fixing mechanism 13 may be any mechanism such as a hook-and-loop fastener, a hook, a connector, a button, or a magnet.

The plurality of electrodes 11 (also referred to as an electrode array) are embedded and fixed in the band 10 such that a contact surface with the living body is exposed to an inner side (living body side) of the band 10. The plurality of electrodes 11 are arranged in a line at equal intervals in the longitudinal direction of the band 10. Thus, when the band 10 is wrapped around the arm, the electrodes 11 come into contact with different circumferential positions of the arm. The number of electrodes 11 is not particularly limited and can be appropriately designed. At least two electrodes 11 (a pair of electrodes) may be provided to measure an ECG signal, and three or more electrodes 11 may be provided to increase the reliability and robustness of measurement. In the present embodiment, a configuration in which six electrodes 11 (three electrode pairs) are provided is adopted.

The electrode 11 is a dry-type metal electrode. The wet-type electrode (gel electrode or the like) has problems such as a possibility of causing skin rash or itching when worn for a long time, and low durability and maintainability, whereas the dry-type electrode 11 does not have such problems. The biosignal measuring device 1 of the present embodiment is assumed to be worn continuously for a long time and to monitor the ECG signal for 24 hours, and thus the electrodes 11 are preferably dry-type electrodes.

The control body 12 is a processing unit that performs control and signal processing of the biosignal measuring device 1. As illustrated in FIG. 4, the control body 12 has a structure in which a measurement circuit 120 for measuring an ECG signal, a biosignal processing device 121 for performing various types of signal processing on the ECG signal, a battery 122, and other circuits are mounted inside a case made of resin or metal, for example. The measurement circuit 120 is an analog circuit including a differential amplifier and the like, and a plurality of electrodes 11 and the measurement circuit 120 constitute an ECG sensor 14. The biosignal processing device 121 includes, for example, an AD converter, a filter, a processor, a memory, and the like. The battery 122 is a rechargeable secondary battery. The control body 12 may be provided with a physical switch, a display, and a notification device that performs notification by sound, light, vibration, electrical stimulation, or the like.

(Control Body)

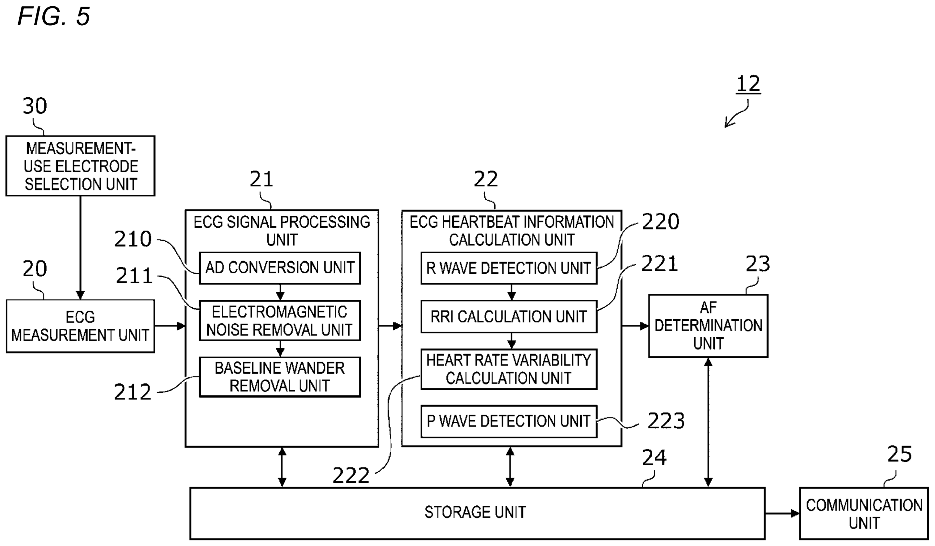

FIG. 5 is a block diagram illustrating an example of a functional configuration of the control body 12.

The control body 12 includes, as a configuration related to the measurement of the ECG signal, a measurement-use electrode selection unit 30, an ECG measurement unit 20, an ECG signal processing unit 21, an ECG heartbeat information calculation unit 22, an AF determination unit 23, a storage unit 24, and a communication unit 25. The measurement-use electrode selection unit 30 selects an electrode pair to be used for measurement of an ECG signal. The ECG measurement unit 20 is a part that amplifies a potential difference between the selected electrode pair by a differential amplifier and outputs the amplified potential difference as an ECG signal. The ECG signal processing unit 21 is a part that performs AD conversion and filtering processing of the ECG signal, and includes an AD conversion unit 210 that performs AD conversion of the ECG signal, an electromagnetic noise removal unit 211 that removes electromagnetic noise from the ECG signal to increase an SN ratio, and a baseline wander removal unit 212 that removes baseline wander (low-frequency wander) of the ECG signal. The ECG heartbeat information calculation unit 22 is a part that extracts various types of heartbeat information from the ECG signal, and includes an R wave detection unit 220, an RRI calculation unit 221, a heart rate variability calculation unit 222, and a P wave detection unit 223. The AF determination unit 23 is a part that detects an occurrence of atrial fibrillation (AF) based on the heartbeat information and calculates an indicator related to AF. The storage unit 24 is a nonvolatile memory that stores measured or calculated data. The communication unit 25 is a part that performs wireless data communication with an external device (for example, a smartphone of a user, another health device, a home server, or the like). Among the functional units illustrated in FIG. 5, the ECG measurement unit 20 is a function provided by the measurement circuit 120 in FIG. 4, and the other functional units are functions provided by the biosignal processing device 121 in FIG. 4.

(ECG Signal Measurement Processing)

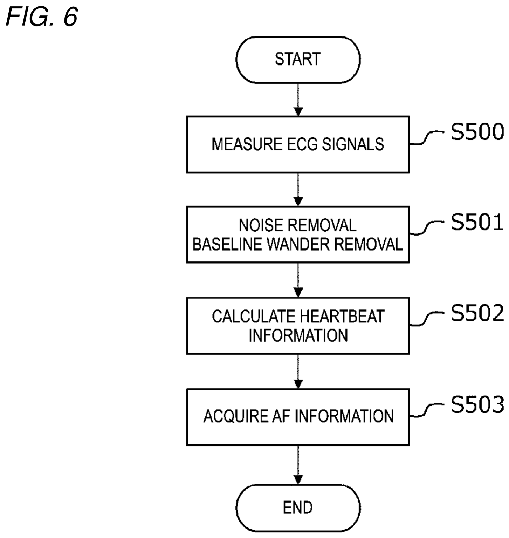

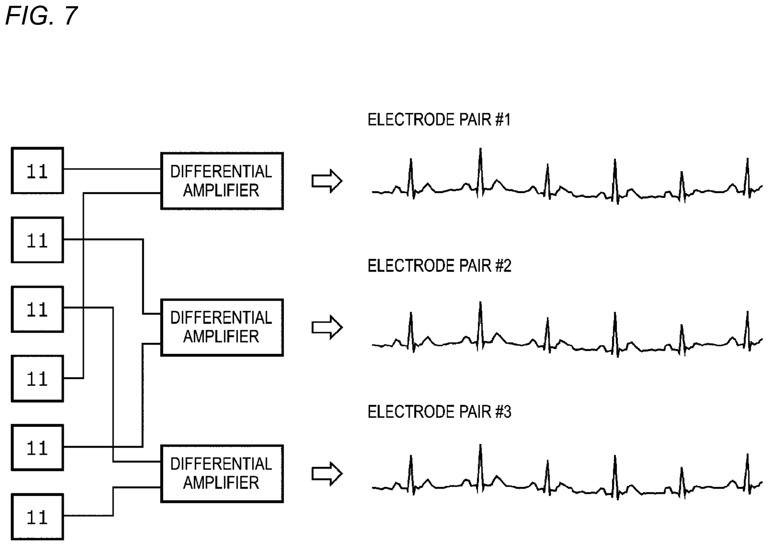

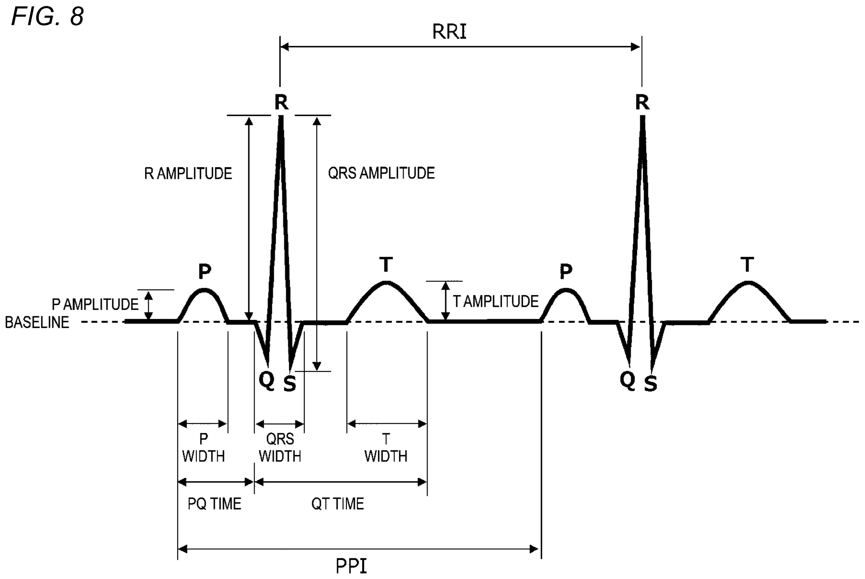

ECG signal measurement processing by the biosignal measuring device 1 will be described with reference to FIGS. 6 to 8. FIG. 6 is a flowchart illustrating a flow of ECG signal measurement processing, FIG. 7 is a diagram illustrating an example of an electrode pair and an ECG signal, and FIG. 8 is a diagram illustrating a waveform of an ECG signal and heartbeat information.

When the user wraps the band 10 around the upper arm, fastens the band 10 with the fixing mechanism 13, and then performs an operation of instructing the start of measurement, the processor of the control body 12 starts the measurement processing of FIG. 6.

In step S500, the ECG measurement unit 20 measures ECG signals of three channels using three electrode pairs. Specifically, the electrode pair (two electrodes 11) to be used for measurement is selected by the measurement-use electrode selection unit 30, and the potential difference between the selected electrode pair is amplified by the differential amplifier and taken in as an ECG signal (analog voltage signal). By sequentially switching the electrode pairs to be selected, ECG signals of three channels are taken in.

In the present embodiment, as illustrated in FIG. 7, three electrode pairs are set such that two electrodes 11 that are just opposite to each other when the band 10 is wrapped around the upper arm are paired. This is because a larger potential difference (i.e., an ECG signal having a higher SN ratio) can be measured when a distance between the two electrodes 11 forming a pair increases. The distance between the two electrodes 11 is, for example, a linear distance between the two electrodes 11 when measured in a state where the band 10 is wrapped around the upper arm as illustrated in FIG. 3. However, the method of setting the electrode pairs is not limited thereto. For example, a multiplexer may be used to freely switch the combination of the electrodes 11 to be paired. In this case, ECG signals of four or more channels can be measured from the six electrodes 11.

In step S501, the ECG signal processing unit 21 performs AD conversion of the ECG signal, and removes electromagnetic noise and baseline wander by digital signal processing. A known noise reduction method such as a band-pass filter, a notch filter, or a moving average can be used to remove electromagnetic noise and baseline wander. The ECG signal (digital data) obtained by the ECG signal processing unit 21 is stored in the storage unit 24 together with information on measurement time.

In step S502, the ECG heartbeat information calculation unit 22 retrieves time-series data of the ECG signal for a unit period from the storage unit 24, and analyzes the time-series data of the ECG signal to calculate various types of heartbeat information. A length of the unit period is arbitrary, and is set to, for example, a length of about several tens of seconds to several tens of minutes.

As schematically illustrated in FIG. 8, the waveform of one heartbeat of the ECG signal mainly includes a P wave, a QRS wave, and a T wave. Note that the QRS wave indicates a waveform obtained by combining an R wave, which is an upward peak, and a downward Q wave and a downward S wave that appear before and after the R wave. In a normal heart, an electrical stimulus generated at the sinoatrial node is transmitted from the atrium to the ventricle, and excitation (contraction) of the atrium and excitation (contraction) of the ventricle occur in sequence, thereby pumping blood. The P wave is a waveform corresponding to the excitation of the atrium, the QRS wave is a waveform corresponding to the excitation of the ventricle, and the T wave is a waveform corresponding to the process of recovery of the ventricle from the excitation. The ECG signal includes a plurality of heartbeat waveforms in a time axis direction. In the case of an average adult, the heart rate is about 60 to 80 bpm, and thus, about 60 to 80 heartbeat waveforms are included in the time-series data of the ECG signal for one minute, for example.

Examples of the heartbeat information include an R amplitude (height of R wave from the baseline), a QRS amplitude (defined by, for example, the sum of the height of R wave from the baseline and the depth of S wave from the baseline), a P amplitude (height of P wave from the baseline), a T amplitude (height of T wave from the baseline), a P width (time from start of P wave to end of P wave), a QRS width (time from start of Q wave to end of S wave), a T width (time from start of T wave to end of T wave), a PQ time (time from start of P wave to start of Q wave), a QT time (time from start of Q wave to end of T wave), an RRI (RR interval; time from peak of R wave to peak of next R wave), a PPI (PP interval; time from start of P wave to start of next P wave), a heart rate variability (temporal variation in RRI and PPI), and the like. It is not necessary to calculate all of the heartbeat information, and only necessary heartbeat information may be calculated. In addition, other heartbeat information may be calculated. The calculated heartbeat information is stored in the storage unit 24 together with the information on the measurement time.

In step S503, the AF determination unit 23 detects the occurrence of AF (atrial fibrillation) based on the heartbeat information, and calculates an indicator related to AF. The AF detection result and the calculated indicator are stored in the storage unit 24 together with the information on the measurement time.

The measurement processing of FIG. 6 described above is repeatedly executed every unit period, whereby the ECG signal and the AF are monitored for 24 hours.

(Details of P Wave Detection Unit)

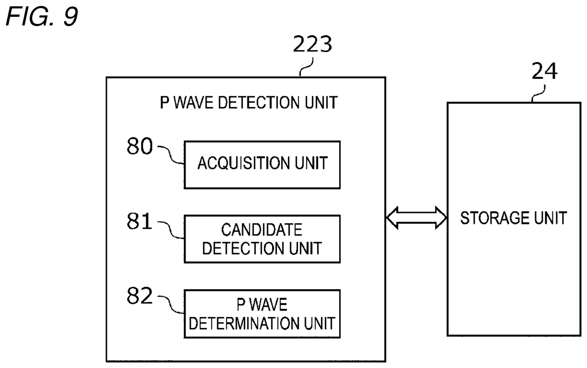

The configuration of the P wave detection unit 223 and the details of a P wave detection algorithm will be described in detail with reference to FIGS. 9 to 12.

FIG. 9 is a functional block diagram of the P wave detection unit 223. The P wave detection unit 223 includes an acquisition unit 80, a candidate detection unit 81, and a P wave determination unit 82 as main functions. The acquisition unit 80 has a function of acquiring the time-series data of the ECG signal from the storage unit 24, the candidate detection unit 81 has a function of detecting a P wave candidate from the time-series data of the ECG signal, and the P wave determination unit 82 has a function of performing detailed determination of P wave presence/absence based on the detection result of the candidate detection unit 81 and outputting a final determination result.

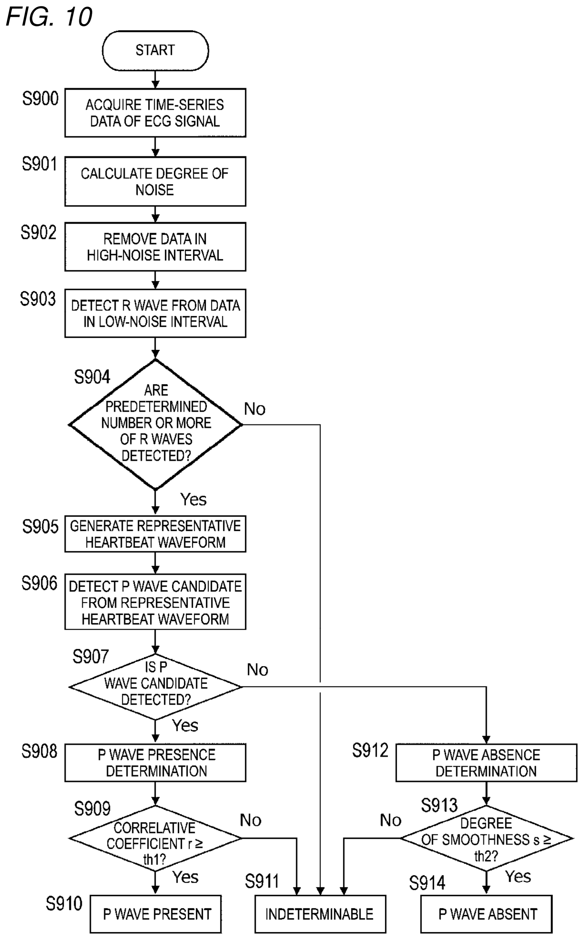

FIG. 10 is a flowchart illustrating a flow of P wave detection processing of P wave detection unit 223.

In step S900, the acquisition unit 80 acquires time-series data of an ECG signal from the storage unit 24. As described above, since the P wave is easily contaminated by noise, it is preferable to perform statistical processing on time-series data in a period of a certain length in order to secure the accuracy and reliability of P wave detection. In the present embodiment, time-series data for ten minutes is used for P wave detection. A period in which the P wave is detected is referred to as a "target period" in the present specification. Note that a length of the target period can be set to any length, and is set to, for example, a length of about several tens of seconds to several tens of minutes.

In step S901, the candidate detection unit 81 calculates the degree of noise (noise level) in the time-series data of the ECG signal in the target period. As the degree of noise, for example, a root mean square (RMS) of the amplitude may be used. In step S902, the candidate detection unit 81 removes data of a high-noise interval (an interval in which the degree of noise is equal to or higher than a predetermined level) from the time-series data of the ECG signal in the target period, and selects data of only a low-noise interval (an interval in which the degree of noise is lower than the predetermined level). By using only data of the low-noise interval, that is, an interval in which a heartbeat waveform appears relatively clearly, for subsequent processing, it is possible to improve the reliability of candidate detection.

In step S903, the candidate detection unit 81 detects an R wave from the data of the low-noise interval. In step S904, the candidate detection unit 81 checks whether a predetermined number or more of R waves (that is, heartbeat waveforms) are detected from the data of the low-noise interval. The "predetermined number" may be set to any number, and may be set to, for example, a value of about 20 to 100 (set to 25 in the present embodiment). If the number of R waves (that is, the number of heartbeat waveforms) extracted from the data of the low-noise interval is less than the predetermined number (No in step S904), the P wave determination unit 82 outputs a result indicating that whether a P wave is included in the ECG signal in the target period is indeterminable (step S911). By allocating the ECG signal to an indeterminable state when the ECG signal has a large amount of noise to the extent that the R wave (heartbeat waveform) is difficult to detect, it is possible to prevent an erroneous determination result from being output in the P wave determination in the subsequent stage, and thus the reliability of the entire device is improved.

If the predetermined number or more of R waves are detected from the data of the low-noise interval (Yes in step S904), the process proceeds to step S905. In step S905, the candidate detection unit 81 extracts, as heartbeat waveforms, waveforms within a predetermined range around the peak position of each detected R wave, and generates a representative heartbeat waveform, which is a typical heartbeat waveform, from a plurality of the extracted heartbeat waveforms. Note that in the present specification, the "typical waveform" refers to a waveform in which a plurality of waveforms are represented by one waveform from the viewpoint of shape. The waveforms may have commonality from the viewpoint of waveform shape, may have commonality when focusing on a specific feature, may have an average shape of a plurality of waveforms, or may be obtained from waveforms excluding exceptional waveforms having significantly different shapes. For example, the waveform may be obtained by averaging a plurality of waveforms, may be a waveform selected as a median value or a mode value when a plurality of waveforms are evaluated by a specific indicator, or may be a waveform generated from a plurality of waveforms using other statistical methods. By generating the "typical waveform", a waveform with further reduced noise can be estimated. As a result, the reliability of the P wave determination can be improved. Similarly, the heartbeat information can be accurately obtained, and the detection accuracy and reliability of atrial fibrillation detection can be improved.

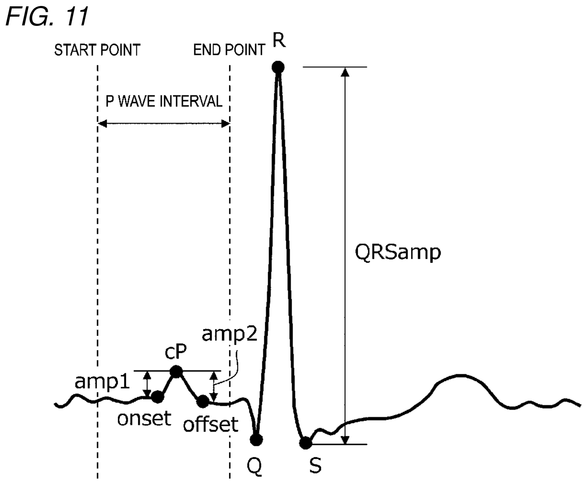

In step S906, the candidate detection unit 81 detects a P wave candidate from the representative heartbeat waveform. FIG. 11 illustrates an example of P wave candidate detection. Specifically, the candidate detection unit 81 first sets a P wave interval in the representative heartbeat waveform with reference to the peak position of the R wave of the representative heartbeat waveform. In the present embodiment, as an example, the definition proposed in NPL 1 is adopted, and the start point and the end point of the P wave interval are set as in the following expressions.

$$\text{Start point} = R - (1 - 0.71) \times RRmedian - 40 \text{ [msec]}$$

$$\text{End point} = R - 0.07 \times RRmedian - 20 \text{ [msec]}$$

R: Peak position of R wave of representative heartbeat waveform
RRmedian: Median value of RRI in target period

However, the start point and the end point of the P wave interval may be determined by other methods without being limited to the above expressions.

Next, the candidate detection unit 81 extracts a feature amount related to the amplitude from the waveform in the P wave interval. For example, as illustrated in FIG. 11, the candidate detection unit 81 selects, as a candidate point cP of the peak of the P wave, the maximum local maximum point or the minimum local minimum point in the P wave interval, and obtains the feature amount of the candidate point cP by the following expression.

[Math. 1]

$$\frac{(amp1 + amp2)/2}{QRSamp}$$

Here, amp1 is an amplitude (peak height) of the candidate point cP relative to a rising point onset of the peak of the candidate point cP, and amp2 is an amplitude (peak height) of the candidate point cP relative to a falling point offset of the peak of the candidate point cP (see FIG. 11). The rising point onset and the falling point offset may be detected by obtaining the inflection point of the waveform. In addition, QRSamp is an amplitude from the minimum point to the maximum point of the QRS wave.

The average of the amplitude amp1 on a rising side and the amplitude amp2 on a falling side is used as the feature amount. This makes it possible to more accurately evaluate the amplitude of the P wave. In addition, since the amplitude of the P wave is divided by the QRS amplitude to be normalized with reference to the QRS amplitude, the magnitude of the amplitude of the P wave can be appropriately evaluated regardless of the strength of the signal level of the ECG signal.

When the feature amount obtained in this manner satisfies a predetermined condition, the candidate detection unit 81 adopts the peak of the candidate point cP as a "P wave candidate" and outputs a result of "P wave candidate present (a P wave candidate is detected)". On the other hand, when the feature amount does not satisfy the predetermined condition, a result of "P wave candidate absent (no P wave candidate is detected)" is output. Based on the knowledge that the amplitude of the P wave is generally a value of about 1% to 5% of the QRS amplitude, the determination condition is set to "$\geq 0.01$" in the present embodiment. That is, when the value of the feature amount is 0.01 or more (in other words, when the amplitude of the P wave interval is 1% or more of the QRS amplitude), a determination of "P wave candidate present" is made.

Since the detection algorithm of the candidate detection unit 81 is only for simply detecting a convex or concave peak, there is a possibility that a noise portion is erroneously detected to be a P wave candidate, or conversely, a P wave buried in noise is missed and erroneously determined to be "P wave candidate absent". Therefore, the P wave determination unit 82 performs detailed determination to make a final determination as to the P wave presence/absence. The P wave determination unit 82 of the present embodiment switches the detailed determination processing according to the detection result of the candidate detection unit 81. That is, if a P wave candidate is detected (Yes in step S907), the "P wave presence determination" (steps S908 to S909) is performed, and if a P wave candidate is not detected (No in step S907), the "P wave absence determination" (steps S912 to S913) is performed. Hereinafter, a specific processing example of each detailed determination will be described.

(1) P Wave Presence Determination

The present inventors have found from the results of analysis of ECG signals obtained in a subject experiment that, although there are individual differences in the waveform of the P wave, a PR interval (an interval between the P wave and the R wave) is kept substantially constant and the waveform of the P wave is reproducible (similar) in the ECG signals of the same subject. Based on this finding, the inventors of the present invention have conceived that, when the P wave interval is set with reference to the time point (for example, the peak position) of the R wave and the waveforms of the P wave intervals in two heartbeat waveforms are compared, the similarity between the two waveforms is high if a P wave appears, but the similarity between the two waveforms is low if it is not the P wave but a noise fluctuation. This idea is embodied by the P wave presence determination algorithm described below.

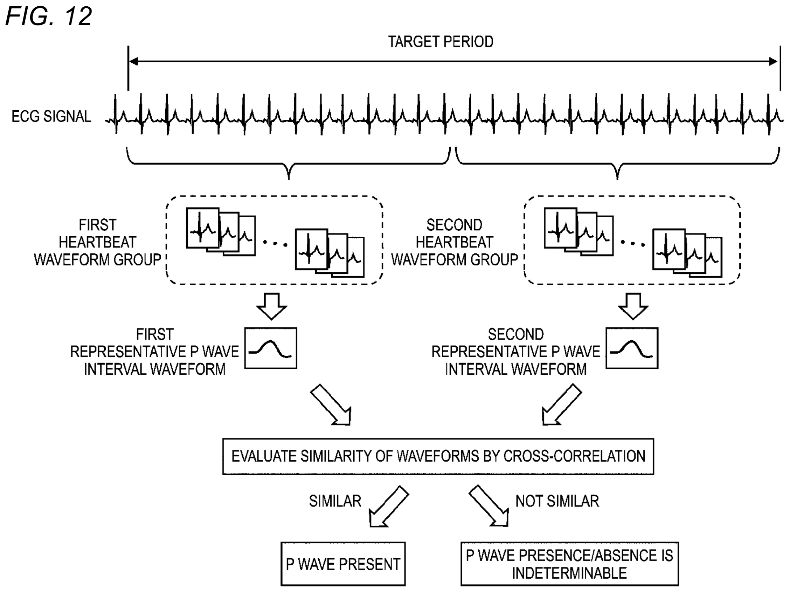

FIG. 12 illustrates an example of an algorithm of the P wave presence determination processing of step S908. The P wave determination unit 82 first extracts a plurality of heartbeat waveforms from the time-series data of the ECG signal in the target period. In this case, the heartbeat waveform may be extracted from the data of the low-noise interval in the same manner as in steps S901 to S905. Alternatively, the data of the heartbeat waveform extracted at step S903 may be stored in the storage unit 24, and the P wave determination unit 82 may use the data for the P wave presence determination. Next, the P wave determination unit 82 divides a plurality of extracted heartbeat waveforms into a first heartbeat waveform group and a second heartbeat waveform group. Here, for example, when N heartbeat waveforms are extracted from the target period, N/2 heartbeat waveforms extracted in the first half are allocated to the first heartbeat waveform group, and N/2 heartbeat waveforms extracted in the second half are allocated to the second heartbeat waveform group. By allocating the same number of heartbeat waveforms to the first heartbeat waveform group and the second heartbeat waveform group, it is possible to make the conditions of the statistical processing in the subsequent stage uniform.

Next, the P wave determination unit 82 acquires a typical P wave interval waveform (referred to as a "first representative P wave interval waveform") from the first heartbeat waveform group. For example, the P wave determination unit 82 may cut out waveforms of P wave intervals set with reference to the time point of the R wave from the heartbeat waveforms of the first heartbeat waveform group, and may set an average of the waveforms as the first representative P wave interval waveform. In this case, as in the case of generating the representative heartbeat waveform at step S905, the waveforms of the plurality of P wave intervals may be averaged, or a median value or a mode value may be obtained from the waveforms of the plurality of P wave intervals. In addition, the P wave determination unit 82 also acquires a typical P wave interval waveform (referred to as a "second representative P wave interval waveform") from the second heartbeat waveform group. Then, the P wave determination unit 82 calculates a cross-correlation coefficient r between the first representative P wave interval waveform and the second representative P wave interval waveform. The cross-correlation coefficient r is an example of an indicator indicating the similarity between the first representative P wave interval waveform and the second representative P wave interval waveform. Note that in the present embodiment, a cross-correlation is used to evaluate the similarity between the first representative P wave interval waveform and the second representative P wave interval waveform, but the similarity may be evaluated using other methods or indicators. For example, the similarity may be evaluated by comparing feature amounts (P amplitude, P width, relative positions of a rising point (onset), a peak position, and a falling point (offset), an area of the P wave, and the like) extracted from each representative P wave interval waveform.

In step S909, the P wave determination unit 82 compares the cross-correlation coefficient r with a predetermined threshold value th1. If the cross-correlation coefficient r is equal to or greater than the threshold value th1 (Yes in step S909), the P wave determination unit 82 determines that the first representative P wave interval waveform and the second representative P wave interval waveform are similar (reproducible), and outputs a result indicating that a P wave is included in the ECG signal in the target period (step S910). On the other hand, if the cross-correlation coefficient r is less than the threshold value th1 (No in step S909), the P wave determination unit 82 determines that the first representative P wave interval waveform and the second representative P wave interval waveform are not similar and the P wave candidate is likely to be noise, and outputs a result indicating that whether the P wave is included in the ECG signal of the target period is indeterminable (step S911). Note that the threshold value th1 may be set to any value, and may be set to a value that enables appropriate separation of P waves and noise, based on the results obtained from subject experiments (in the present embodiment, the threshold value th1 is set to 0.7).

(2) P Wave Absence Determination

Even if the P wave candidate is not detected by the candidate detection unit 81, there is a possibility that the P wave is missed due to being buried in noise. In this regard, the P wave determination unit 82 evaluates degree of smoothness of the P wave interval of the heartbeat waveform to determine whether the detection result of the candidate detection unit 81 may be adopted. If the degree of smoothness is high, the noise level of the ECG signal can be regarded as low (the SN ratio is high), and therefore, it can be determined that the reliability of the detection result of the candidate detection unit 81 is high. Conversely, if the degree of smoothness of the P wave interval is low, the noise level of the ECG signal can be regarded as high (the SN ratio is low), and therefore, it can be determined that the reliability of the detection result of the candidate detection unit 81 is low.

In the present embodiment, a degree of smoothness s of the P wave interval is indexed as in the following expression.

Degree of smoothness s = (QRS amplitude) / (RMS value of numerical differentiation of waveform in P wave interval)

The denominator of the indicator (the RMS value of the numerical differentiation of the waveform in the P wave interval) represents a magnitude of a change (unevenness) in the waveform in the P wave interval. Therefore, the smaller (flatter) the change in the waveform of the P wave interval, the smaller the degree of smoothness. In addition, since the indicator is standardized with reference to the QRS amplitude, the degree of smoothness of the P wave interval can be appropriately evaluated regardless of the strength of the signal level of the ECG signal.

To be specific, the P wave determination unit 82 calculates the degree of smoothness s of the P wave interval by using the representative heartbeat waveform generated in step S905 (step S912). Then, the P wave determination unit 82 compares the degree of smoothness s with a predetermined threshold value th2. If the degree of smoothness s is equal to or higher than the threshold value th2 (Yes in step S913), the P wave determination unit 82 determines that the reliability of the detection result of the candidate detection unit 81 is high, and outputs a result indicating that the P wave is not included in the ECG signal in the target period (step S914). On the other hand, if the degree of smoothness s is less than the threshold value th2 (No in step S913), the P wave determination unit 82 determines that the reliability of the detection result of the candidate detection unit 81 is low, and outputs a result indicating that whether the P wave is included in the ECG signal of the target period is indeterminable (step S911). Note that the threshold value th2 may be set to any value, and may be set to a value that enables appropriate separation of noise, based on the results obtained by subject experiments (in the present embodiment, the threshold value th2 is set to about 800).

According to the P wave detection algorithm of the present embodiment described above, the P wave presence/absence can be determined with high reliability from the time-series data of the ECG signal. Therefore, it is possible to automatically and accurately detect a disease such as atrial fibrillation.

<Modified Example>

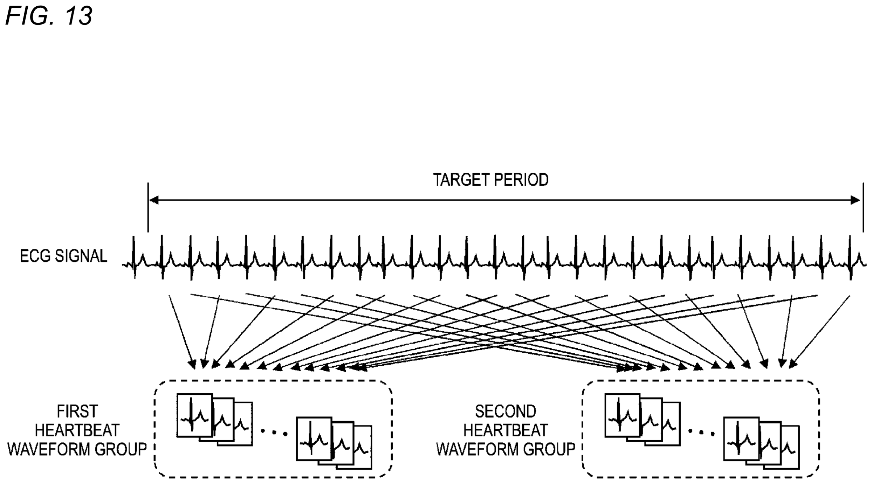

In the above embodiment, as illustrated in FIG. 12, the target period is divided into two periods, and the first heartbeat waveform group is extracted from the first half and the second heartbeat waveform group is extracted from the second half. However, the method of allocation to the first heartbeat waveform group and the second heartbeat waveform group is not limited to this method. For example, as illustrated in FIG. 13, a plurality of heartbeat waveforms extracted from the time-series data of the ECG signal may be alternately allocated to the first heartbeat waveform group and the second heartbeat waveform group (that is, odd-numbered heartbeat waveforms are allocated to the first heartbeat waveform group and even-numbered heartbeat waveforms are allocated to the second heartbeat waveform group). Alternatively, the heartbeat waveforms may be randomly allocated to the first heartbeat waveform group and the second heartbeat waveform group, instead of being regularly allocated alternately.

In the case of time division as illustrated in FIG. 12, the extraction period of the first heartbeat waveform group and the extraction period of the second heartbeat waveform group do not overlap, whereas in the case of alternate or random allocation, both the first heartbeat waveform group and the second heartbeat waveform group are extracted from the entire target period.

For example, in a case where a temporal change appears in the waveform of the P wave, such as a case where the direction (polarity) of the P wave is inverted between the first half and the second half of the target period, in the method of FIG. 12, the cross-correlation coefficient between the first representative P wave interval waveform and the second representative P wave interval waveform becomes small, and there is a possibility that a determination of "indeterminable" is made even though the P wave is present. In contrast, in the case of alternate or random allocation, both the first heartbeat waveform group and the second heartbeat waveform group are extracted from the entire target period, and thus, the temporal change in the waveform of the P wave similarly affects both the first representative P wave interval waveform and the second representative P wave interval waveform, which can suppress the reduction of the cross-correlation coefficient. That is, the P wave presence determination according to the modified example has an advantage of being robust against a temporal change in the waveform of the P wave.

<Others>

The embodiments described above are merely illustrative of configuration examples of the present invention. The present invention is not limited to the specific aspects described above, and various modifications are possible within the scope of the technical idea of the present invention. For example, as the fixing member for fixing the electrodes 11 in a state in which the electrodes are pressed against the living body, a band-shaped (belt-shaped) member that is wrapped around the measurement portion of the living body in a state in which the electrodes 11 are brought into contact, as in the above embodiments, an envelope-shaped member that covers and wraps the measurement portion of the living body, or a ring-shaped member may be used. Additionally, the fixing member may have elasticity or deformability in order to correspond to the size (diameter) of the measurement portion of the living body. Alternatively, when the fixing member is made of a non-elastic material, the fixing member may have a structure that allows adjustment of the length. The number of electrodes 11 may be one or more. The arrangement of the plurality of electrodes 11 is not necessarily one line, and the electrodes 11 may be arranged in a two-dimensional array. In addition, the electrodes 11 may be arranged at irregular intervals, instead of at equal intervals. The electrode 11 may be integrated with the fixing member (band 10), or may have a separate structure from the fixing member. The control body 12 need not be fixed to the band 10. For example, the control body 12 and the band 10 may be formed as separate structures, and the control body 12 and the band 10 (the electrode 11) may be connected to each other by a cable.

In the P wave presence determination of the above embodiment, a representative P wave interval waveform is generated based on a waveform group of a plurality of P wave intervals extracted from a plurality of heartbeat waveforms, and the presence of the P wave is determined by evaluating a similarity between two representative P wave interval waveforms. However, it is not essential to use the representative P wave interval waveform in the P wave presence determination, and a P wave interval waveform extracted from a single heartbeat waveform may be used for the evaluation of the similarity. In this case, it is only necessary to evaluate whether the similarity between a waveform of a first P wave interval set with reference to a time point of a first R wave in the time-series data of the ECG signal and a waveform of a second P wave interval set with reference to a time point of a second R wave different from the first R wave is higher than a threshold value. In addition, in the above embodiment, the plurality of heartbeat waveforms extracted from the time-series data of the ECG signal are divided into two groups, the first heartbeat waveform group and the second heartbeat waveform group, but may be divided into three or more groups to be compared with each other, for example. Alternatively, the plurality of heartbeat waveforms may be divided into three groups such as the first heartbeat waveform group, the second heartbeat waveform group, and a heartbeat waveform group to be excluded from the similarity evaluation.

The present specification includes the following disclosures.

[Supplement 1]

A biosignal processing device (121) including:

an acquisition unit (80) that acquires time-series data of an ECG signal measured from a user; and
a P wave determination unit (82) that extracts a first P wave interval waveform from a first P wave interval set with reference to a time point of a first R wave in the time-series data of the ECG signal and extracts a second P wave interval waveform from a second P wave interval set with reference to a time point of a second R wave different from the first R wave, and when a similarity between the first P wave interval waveform and the second P wave interval waveform is higher than a threshold value, determines that a P wave is included in the first P wave interval and the second P wave interval.

[Supplement 2]

A biosignal processing device (121) including:

an acquisition unit (80) that acquires time-series data of an ECG signal measured from a user; and
a P wave determination unit (82) that extracts at least a first heartbeat waveform group and a second heartbeat waveform group from the time-series data of the ECG signal, extracts waveforms of P wave intervals each set with reference to a time point of an R wave from respective heartbeat waveforms of the first heartbeat waveform group and extracts waveforms of P wave intervals each set with reference to a time point of an R wave from respective heartbeat waveforms of the second heartbeat waveform group, generates a first representative P wave interval waveform, based on a waveform group of a plurality of P wave intervals extracted from the first heartbeat waveform group and generates a second representative P wave interval waveform, based on a waveform group of a plurality of P wave intervals extracted from the second heartbeat waveform group, and when a similarity between the first representative P wave interval waveform and the second representative P wave interval waveform is higher than a threshold value, determines that a P wave is included in the first heartbeat waveform group and the second heartbeat waveform group.

[Supplement 3]

A biosignal processing device (121) including:

an acquisition unit (80) that acquires time-series data of an ECG signal measured from a user;
a candidate detection unit (81) that detects a P wave candidate from the time-series data of the ECG signal; and
a P wave determination unit (82) that, in a case where the P wave candidate is detected by the candidate detection unit (81), divides a plurality of heartbeat waveforms extracted from the time-series data of the ECG signal into a plurality of heartbeat waveform groups including at least a first heartbeat waveform group and a second heartbeat waveform group, acquires a first representative P wave interval waveform and a second representative P wave interval waveform, which are typical P wave interval waveforms, from the first heartbeat waveform group and the second heartbeat waveform group, respectively, and when a similarity between the first representative P wave interval waveform and the second representative P wave interval waveform is higher than a threshold value, determines that a P wave is included in the time-series data of the ECG signal.

[Supplement 4]

The biosignal processing device according to supplement 3, in which
when the similarity between the first representative P wave interval waveform and the second representative P wave interval waveform is equal to or less than the threshold value, the P wave determination unit determines that whether the P wave is included in the time-series data of the ECG signal is indeterminable.

[Supplement 5]

The biosignal processing device according to supplement 3 or 4, in which
the P wave determination unit allocates, among the plurality of heartbeat waveforms extracted from the time-series data of the ECG signal, a heartbeat waveform extracted in a first half into the first heartbeat waveform group and a heartbeat waveform extracted in a second half into the second heartbeat waveform group.

[Supplement 6]

The biosignal processing device according to supplement 3 or 4, in which
the P wave determination unit alternately or randomly selects, from among the plurality of heartbeat waveforms extracted from the time-series data of the ECG signal, a heartbeat waveform to be allocated to the first heartbeat waveform group and a heartbeat waveform to be allocated to the second heartbeat waveform group.

[Supplement 7]

The biosignal processing device according to any one of supplements 3 to 6, in which the P wave determination unit divides the plurality of heartbeat waveforms extracted from the time-series data of the ECG signal into the first heartbeat waveform group and the second heartbeat waveform group such that the number of heartbeat waveforms of the first heartbeat waveform group and the number of heartbeat waveforms of the second heartbeat waveform group are equal to each other.

[Supplement 8]

The biosignal processing device according to any one of supplements 3 to 7, in which the P wave determination unit

extracts waveforms of P wave intervals each set with reference to a time point of an R wave from respective heartbeat waveforms of the first heartbeat waveform group, and acquires, as the first representative P wave interval waveform, an average, a median value, or a mode value of waveforms of a plurality of P wave intervals extracted from the first heartbeat waveform group, and
extracts waveforms of P wave intervals each set with reference to a time point of an R wave from respective heartbeat waveforms of the second heartbeat waveform group, and acquires, as the second representative P wave interval waveform, an average, a median value, or a mode value of waveforms of a plurality of P wave intervals extracted from the second heartbeat waveform group.

[Supplement 9]

The biosignal processing device according to any one of supplements 3 to 8, in which the P wave determination unit evaluates the similarity between the first representative P wave interval waveform and the second representative P wave interval waveform by cross-correlation between the first representative P wave interval waveform and the second representative P wave interval waveform.

[Supplement 10]

The biosignal processing device according to any one of supplements 3 to 9, in which the candidate detection unit generates a representative heartbeat waveform, which is a typical heartbeat waveform, from a plurality of heartbeat waveforms extracted from the time-series data of the ECG signal, and detects a P wave candidate from the representative heartbeat waveform.

[Supplement 11]

The biosignal processing device according to supplement 10, in which
the candidate detection unit extracts a feature amount from a P wave interval set with reference to a time point of an R wave in the representative heartbeat waveform, and when the feature amount satisfies a predetermined condition, determines that the P wave candidate is present.

[Supplement 12]

The biosignal processing device according to supplement 10 or 11, in which
the candidate detection unit selects, from the time-series data of the ECG signal, data of a low-noise interval in which a degree of noise is lower than a predetermined level, and generates the representative heartbeat waveform by using a plurality of heartbeat waveforms extracted from the data of the low-noise interval.

[Supplement 13]

The biosignal processing device according to supplement 12, in which
when the number of heartbeat waveforms extracted from the data of the low-noise interval is less than a predetermined number, the P wave determination unit determines that whether the P wave is included in the time-series data of the ECG signal is indeterminable.

[Supplement 14]

The biosignal processing device according to any one of supplements 10 to 13, in which when the P wave candidate is not detected by the candidate detection unit, the P wave determination unit evaluates a degree of smoothness of a P wave interval set with reference to a time point of an R wave in the representative heartbeat waveform, determines, in a case where the degree of smoothness is equal to or greater than a predetermined value, that the P wave is not included in the time-series data of the ECG signal, and determines, in other cases, that whether the P wave is included in the time-series data of the ECG signal is indeterminable.

[Supplement 15]

A biosignal measuring device (1) including:

an ECG sensor (14) that is configured to be worn on a limb of a user; and
the biosignal processing device (12) according to any one of supplements 1 to 14,
which processes an ECG signal measured by the ECG sensor (14).

[Supplement 16]

A biosignal processing method performed by a biosignal processing device (121), the biosignal processing method including:

a step of acquiring time-series data of an ECG signal measured from a user; and
a step of extracting a first P wave interval waveform from a first P wave interval set with reference to a time point of a first R wave in the time-series data of the ECG signal and extracting a second P wave interval waveform from a second P wave interval set with reference to a time point of a second R wave different from the first R wave, and when a similarity between the first P wave interval waveform and the second P wave interval waveform is higher than a threshold value, determining that a P wave is included in the first P wave interval and the second P wave interval.

[Supplement 17]

A biosignal processing method performed by a biosignal processing device (121), the biosignal processing method including:

a step of acquiring time-series data of an ECG signal measured from a user; and
steps of extracting at least a first heartbeat waveform group and a second heartbeat waveform group from the time-series data of the ECG signal, extracting waveforms of P wave intervals each set with reference to a time point of an R wave from respective heartbeat waveforms of the first heartbeat waveform group and extracting waveforms of P wave intervals set with reference to a time point of an R wave from respective heartbeat waveforms of the second heartbeat waveform group, generating a first representative P wave interval waveform, based on a waveform group of a plurality of P wave intervals extracted from the first heartbeat waveform group and generating a second representative P wave interval waveform, based on a waveform group of a plurality of P wave intervals extracted from the second heartbeat waveform group, and when a similarity between the first representative P wave interval waveform and the second representative P wave interval waveform is higher than a threshold value, determining that a P wave is included in the first heartbeat waveform group and the second heartbeat waveform group.

[Supplement 18]

A biosignal processing method performed by a biosignal processing device (12), the biosignal processing method including:

a step (S900) of acquiring time-series data of an ECG signal measured from a user;
a step (S906) of detecting a P wave candidate from the time-series data of the ECG signal; and
steps (S908 to S911) of, in a case where the P wave candidate is detected, dividing a plurality of heartbeat waveforms extracted from the time-series data of the ECG signal into a plurality of heartbeat waveform groups including at least a first heartbeat waveform group and a second heartbeat waveform group, acquiring a first representative P wave interval waveform and a second representative P wave interval waveform, which are typical P wave interval waveforms, from the first heartbeat waveform group and the second heartbeat waveform group, respectively, and when a similarity between the first representative P wave interval waveform and the second representative P wave interval waveform is higher than a threshold value, determining that a P wave is included in the time-series data of the ECG signal.

REFERENCE SIGNS

1: Biosignal measuring device
10: Band
11: Electrode
12: Control body
14: ECG sensor
121: Biosignal processing device

**Claims**

1.  A biosignal processing device comprising:

an acquisition unit that acquires time-series data of an ECG signal measured from a user; and
a P wave determination unit that extracts a first P wave interval waveform from a first P wave interval set with reference to a time point of a first R wave in the time-series data of the ECG signal and extracts a second P wave interval waveform from a second P wave interval set with reference to a time point of a second R wave different from the first R wave, and when a similarity between the first P wave interval waveform and the second P wave interval waveform is higher than a threshold value, determines that a P wave is included in the first P wave interval and the second P wave interval.

2.  A biosignal processing device comprising:

an acquisition unit that acquires time-series data of an ECG signal measured from a user; and
a P wave determination unit that extracts at least a first heartbeat waveform group and a second heartbeat waveform group from the time-series data of the ECG signal, extracts waveforms of P wave intervals each set with reference to a time point of an R wave from respective heartbeat waveforms of the first heartbeat waveform group and extracts waveforms of P wave intervals each set with reference to a time point of an R wave from respective heartbeat waveforms of the second heartbeat waveform group, generates a first representative P wave interval waveform, based on a waveform group of a plurality of P wave intervals extracted from the first heartbeat

15

waveform group and generates a second representative P wave interval waveform, based on a waveform group of a plurality of P wave intervals extracted from the second heartbeat waveform group, and when a similarity between the first representative P wave interval waveform and the second representative P wave interval waveform is higher than a threshold value, determines that a P wave is included in the first heartbeat waveform group and the second heartbeat waveform group.

3. A biosignal processing device comprising:

an acquisition unit that acquires time-series data of an ECG signal measured from a user;
a candidate detection unit that detects a P wave candidate from the time-series data of the ECG signal; and
a P wave determination unit that, in a case where the P wave candidate is detected by the candidate detection unit, divides a plurality of heartbeat waveforms extracted from the time-series data of the ECG signal into a plurality of heartbeat waveform groups including at least a first heartbeat waveform group and a second heartbeat waveform group, acquires a first representative P wave interval waveform and a second representative P wave interval waveform, which are typical P wave interval waveforms, from the first heartbeat waveform group and the second heartbeat waveform group, respectively, and when a similarity between the first representative P wave interval waveform and the second representative P wave interval waveform is higher than a threshold value, determines that a P wave is included in the time-series data of the ECG signal.

4. The biosignal processing device according to claim 3, wherein
when the similarity between the first representative P wave interval waveform and the second representative P wave interval waveform is equal to or less than the threshold value, the P wave determination unit determines that whether the P wave is included in the time-series data of the ECG signal is indeterminable.

5. The biosignal processing device according to claim 3, wherein
the P wave determination unit allocates, among the plurality of heartbeat waveforms extracted from the time-series data of the ECG signal, a heartbeat waveform extracted in a first half into the first heartbeat waveform group and a heartbeat waveform extracted in a second half into the second heartbeat waveform group.

6. The biosignal processing device according to claim 3, wherein
the P wave determination unit alternately or randomly selects, from among the plurality of heartbeat waveforms extracted from the time-series data of the ECG signal, a heartbeat waveform to be allocated to the first heartbeat waveform group and a heartbeat waveform to be allocated to the second heartbeat waveform group.

7. The biosignal processing device according to claim 3, wherein
the P wave determination unit divides the plurality of heartbeat waveforms extracted from the time-series data of the ECG signal into the first heartbeat waveform group and the second heartbeat waveform group such that the number of heartbeat waveforms of the first heartbeat waveform group and the number of heartbeat waveforms of the second heartbeat waveform group are equal to each other.

8. The biosignal processing device according to claim 3, wherein

the P wave determination unit
extracts waveforms of P wave intervals each set with reference to a time point of an R wave from respective heartbeat waveforms of the first heartbeat waveform group, and acquires, as the first representative P wave interval waveform, an average, a median value, or a mode value of waveforms of a plurality of P wave intervals extracted from the first heartbeat waveform group, and
extracts waveforms of P wave intervals each set with reference to a time point of an R wave from respective heartbeat waveforms of the second heartbeat waveform group, and acquires, as the second representative P wave interval waveform, an average, a median value, or a mode value of waveforms of a plurality of P wave intervals extracted from the second heartbeat waveform group.

9. The biosignal processing device according to claim 3, wherein
the P wave determination unit evaluates the similarity between the first representative P wave interval waveform and the second representative P wave interval waveform by cross-correlation between the first representative P wave interval waveform and the second representative P wave interval waveform.

10. The biosignal processing device according to claim 3, wherein

the candidate detection unit generates a representative heartbeat waveform, which is a typical heartbeat waveform, from a plurality of heartbeat waveforms extracted from the time-series data of the ECG signal, and detects a P wave candidate from the representative heartbeat waveform.

11. The biosignal processing device according to claim 10, wherein
the candidate detection unit extracts a feature amount from a P wave interval set with reference to a time point of an R wave in the representative heartbeat waveform, and when the feature amount satisfies a predetermined condition, determines that the P wave candidate is present.

12. The biosignal processing device according to claim 10, wherein
the candidate detection unit selects, from the time-series data of the ECG signal, data of a low-noise interval in which a degree of noise is lower than a predetermined level, and generates the representative heartbeat waveform by using a plurality of heartbeat waveforms extracted from the data of the low-noise interval.

13. The biosignal processing device according to claim 12, wherein
when the number of heartbeat waveforms extracted from the data of the low-noise interval is less than a predetermined number, the P wave determination unit determines that whether the P wave is included in the time-series data of the ECG signal is indeterminable.

14. The biosignal processing device according to claim 10, wherein
when the P wave candidate is not detected by the candidate detection unit, the P wave determination unit evaluates a degree of smoothness of a P wave interval set with reference to a time point of an R wave in the representative heartbeat waveform, determines, in a case where the degree of smoothness is equal to or greater than a predetermined value, that the P wave is not included in the time-series data of the ECG signal, and determines, in other cases, that whether the P wave is included in the time-series data of the ECG signal is indeterminable.

15. A biosignal measuring device comprising:

an ECG sensor that is configured to be worn on a limb of a user; and
the biosignal processing device according to any one of claims 1 to 14, which processes an ECG signal measured by the ECG sensor.

16. A biosignal processing method performed by a biosignal processing device, the biosignal processing method comprising:

acquiring time-series data of an ECG signal measured from a user; and
extracting a first P wave interval waveform from a first P wave interval set with reference to a time point of a first R wave in the time-series data of the ECG signal and extracting a second P wave interval waveform from a second P wave interval set with reference to a time point of a second R wave different from the first R wave, and when a similarity between the first P wave interval waveform and the second P wave interval waveform is higher than a threshold value, determining that a P wave is included in the first P wave interval and the second P wave interval.

17. A biosignal processing method performed by a biosignal processing device, the biosignal processing method comprising:

acquiring time-series data of an ECG signal measured from a user; and
extracting at least a first heartbeat waveform group and a second heartbeat waveform group from the time-series data of the ECG signal, extracting waveforms of P wave intervals each set with reference to a time point of an R wave from respective heartbeat waveforms of the first heartbeat waveform group and extracting waveforms of P wave intervals each set with reference to a time point of an R wave from respective heartbeat waveforms of the second heartbeat waveform group, generating a first representative P wave interval waveform, based on a waveform group of a plurality of P wave intervals extracted from the first heartbeat waveform group and generating a second representative P wave interval waveform, based on a waveform group of a plurality of P wave intervals extracted from the second heartbeat waveform group, and when a similarity between the first representative P wave interval waveform and the second representative P wave interval waveform is higher than a threshold value, determining that a P wave is included in the first heartbeat waveform group and the second heartbeat waveform group.

18. A biosignal processing method performed by a biosignal processing device, the biosignal processing method comprising:

acquiring time-series data of an ECG signal measured from a user;

detecting a P wave candidate from the time-series data of the ECG signal; and

in a case where the P wave candidate is detected, dividing a plurality of heartbeat waveforms extracted from the time-series data of the ECG signal into a plurality of heartbeat waveform groups including at least a first heartbeat waveform group and a second heartbeat waveform group, acquiring a first representative P wave interval waveform and a second representative P wave interval waveform, which are typical P wave interval waveforms, from the first heartbeat waveform group and the second heartbeat waveform group, respectively, and when a similarity between the first representative P wave interval waveform and the second representative P wave interval waveform is higher than a threshold value, determining that a P wave is included in the time-series data of the ECG signal.

EP 4 736 768 A1

## FIG. 1

ECG SIGNAL

EVALUATION OF IRREGULARITY OF HEARTBEAT INTERVAL
DETERMINATION OF P WAVE PRESENCE/ABSENCE

CONSTANT MONITORING OF ATRIAL FIBRILLATION
RECORDING OF AF burden

FIG. 2

FIG. 3

*FIG. 4*

1

14

11

11

11

11

11

11

120

12

121

122

MEASUREMENT CIRCUIT

BIOSIGNAL PROCESSING DEVICE

BATTERY

FIG. 5

12

30 MEASUREMENT-USE ELECTRODE SELECTION UNIT

20 ECG MEASUREMENT UNIT

21 ECG SIGNAL PROCESSING UNIT
- 210 AD CONVERSION UNIT
- 211 ELECTROMAGNETIC NOISE REMOVAL UNIT
- 212 BASELINE WANDER REMOVAL UNIT

22 ECG HEARTBEAT INFORMATION CALCULATION UNIT
- 220 R WAVE DETECTION UNIT
- 221 RRI CALCULATION UNIT
- 222 HEART RATE VARIABILITY CALCULATION UNIT
- 223 P WAVE DETECTION UNIT

23 AF DETERMINATION UNIT

24 STORAGE UNIT

25 COMMUNICATION UNIT

FIG. 6

START

MEASURE ECG SIGNALS — S500

NOISE REMOVAL
BASELINE WANDER REMOVAL — S501

CALCULATE HEARTBEAT
INFORMATION — S502

ACQUIRE AF INFORMATION — S503

END

## FIG. 7

ELECTRODE PAIR #1

ELECTRODE PAIR #2

ELECTRODE PAIR #3

EP 4 736 768 A1

FIG. 8

FIG. 9

223

P WAVE DETECTION UNIT

80 — ACQUISITION UNIT

81 — CANDIDATE DETECTION UNIT

82 — P WAVE DETERMINATION UNIT

24

STORAGE UNIT

## FIG. 10

FIG. 11

FIG. 12

TARGET PERIOD

ECG SIGNAL

FIRST HEARTBEAT WAVEFORM GROUP

SECOND HEARTBEAT WAVEFORM GROUP

FIRST REPRESENTATIVE P WAVE INTERVAL WAVEFORM

SECOND REPRESENTATIVE P WAVE INTERVAL WAVEFORM

EVALUATE SIMILARITY OF WAVEFORMS BY CROSS-CORRELATION

SIMILAR

NOT SIMILAR

P WAVE PRESENT

P WAVE PRESENCE/ABSENCE IS INDETERMINABLE

EP 4 736 768 A1

FIG. 13

## INTERNATIONAL SEARCH REPORT

| International application No. |
|---|
| **PCT/JP2024/028015** |

**A. CLASSIFICATION OF SUBJECT MATTER**

*A61B 5/353*(2021.01)i
FI: A61B5/353

According to International Patent Classification (IPC) or to both national classification and IPC

**B. FIELDS SEARCHED**

Minimum documentation searched (classification system followed by classification symbols)

A61B5/24-5/367

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | US 5840038 A (MARQUETTE MEDICAL SYSTEMS, INC.) 24 November 1998 (1998-11-24) entire text, all drawings | 1-18 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **19 September 2024** | **01 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/028015**

| Patent document cited in search report | | | Publication date (day/month/year) | Patent family member(s) | Publication date (day/month/year) |
|---|---|---|---|---|---|
| US | 5840038 | A | 24 November 1998 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5840038 A

**Non-patent literature cited in the description**

- **SACLOVA, L. ; NEMCOVA, A. ; SMISEK, R. et al.** Reliable P wave detection in pathological ECG signals. *Sci Rep*, 2022, vol. 12, 6589